# EUROPEAN PATENT SPECIFICATION

(11) **EP 1 200 317 B1**
(45) Date of publication and mention of the grant of the patent: **18.06.2003**
(21) Application number: 00947999.9
(22) Date of filing: 26.07.2000
(51) Int. Cl.: B65D 77/06, A61K 7/48, A61K 7/00, B65D 83/14

(54) **A DEVICE FOR SPRAY DISPENSING A COMPOSITION FOR TOPICAL APPLICATION COMPRISING VITAMIN E AND ESSENTIAL FATTY ACIDS**
ZERSTÄUBER ZUR TOPISCHEN ANWENDUNG EINER ZUBEREITUNG, DIE VITAMIN E UND ESSENTIELLE FETTSÄUREN ENTHÄLT
DISPOSITIF DE DISTRIBUTION D'UNE COMPOSITION PAR PULVERISATION POUR APPLICATION TOPIQUE CONTENANT DE LA VITAMINE E ET DES ACIDES GRAS ESSENTIELS

(30) Priority: 03.08.1999 IT MI991747
(43) Date of publication of application: 02.05.2002
(73) Proprietor: BIO.LO.GA. S.r.l., 31015 Conegliano (TV) (IT)
(72) Inventor: Panin, Giorgio, 45100 Rovigo (IT)
(74) Representative: Ferreccio, Rinaldo
(86) International application number: EP0007168
(87) International publication number: WO01009000

(56) References cited:
- EP-A- 0 711 540
- FR-A- 2 436 085

## Description

In its broader aspect, the present invention relates to the field of dermatology and dermocosmesis.

More in particular, the invention relates to a device that is capable of dispensing in the form of spray a composition based on vitamin E and essential fatty acids.

It is known that both vitamin E and essential fatty acids exert a beneficial action at the skin; in particular, vitamin E, i.e. α-tocopherol and derivatives thereof are substances which are widely used in the cosmetic industry, by virtue of their properties as antioxidants and free radicals scavengers, in the preparation of formulations for combating or preventing unsightly skin conditions or for slowing down the cellular aging. As it is reported in US 4 144 325, α-tocopherol is also capable of protecting the skin from UV radiation, as it absorbs in the 295-315 nm wavelength range, thereby preventing the formation of erythema due to exposure to the sunlight or to artificial UV sources. However, free α-tocopherol is photolabile and oxidizable in presence of air and the compositions that contain it do not show enough stability to storage.

Essential fatty acids (EFA) are fatty acids that cannot be synthesized by human beings and therefore need to be introduced as part of the alimentary diet in order to ensure the good health of the body. The essential fatty acids in the vertebrates and consequently in the human beings are linoleic acid, from which derives the Omega-6 (Ω-6) series (γ-linolenic acid, dihomo-γ-linolenic acid and arachidonic acid) and the α-linoleic acid, from which derives the Omega-3 (Ω-3) series (eicosapentaenoic and docosahexaenoic acid).

The Ω-6 and Ω-3 acids are polyunsaturated fatty acids, also known as PUFA (Polyunsaturated Fatty Acids) which can be taken with the alimentary diet although vertebrates may also synthesize them starting from essential fatty acids.

The linoleic acid, in particular, plays a fundamental role in the normal constitution of the interlamellar lipidic component of the horny layer of epidermis and hence in the correct barrier function of the horny layer. In fact, it represents about 50 % of the esterified fatty acids in the ceramides of the lamellar bodies and the ceramides are the most important class of the interlamellar lipids. Indeed, the ceramides build up a structure of single o double lipid layers into which the other classes of interlamellar lipids do insert themselves, i.e. the aliphatic chains of the free fatty acids and of cholesterol.

The interlamellar lipids bond also to the protein envelope of the horny layer cells, thus forming a coating having a cementing function and a function of selective permeability, contributing in an important way to provide the skin with compactness, elasticity and plasticity.

Through the application to skin of linoleic acid, the most important of all the essential fatty acids, it has been observed a benefit in the control of skin dryness and of transepidermal loss of water.

Further on, it has been proposed the use of essential fatty acids in the treatment of atopic dermatitis and of acne.

However, as they are polyunsaturated, the essential fatty acids are unfortunately easily oxidizable in the presence of air and light, what jeopardizes the shelf-life of pharmaceutical o cosmetic compositions containing them.

The problem underlying the present invention is that of providing a composition for topical use containing both vitamin E and essential fatty acids (EFA) or Ω-6- or Ω-3-polyunsaturated fatty acids, that has satisfying storability properties.

According to the present invention, such a problem has been solved by a device for spray delivering a composition for topical application comprising a collapsible container which in turn is enclosed in a spray bomb filled with pressurized fluid, that causes said composition to be dispensed in finely atomized form, characterized in that said composition comprises a low vapor pressure vehicle consisting of one or more volatile silicones, one or more essential (EFA) or polyunsaturated Ω-6 or Ω-3 (PUFA) fatty acids or one or more oils that contain them and vitamin E.

Preferably, the volatile silicones are chosen among pentamer cyclomethicone, tetramer cyclomethicone, hexamethyldisiloxane and mixtures thereof. The use of pentamer cyclomethicone is particularly preferred.

Nitrogen, carbon dioxide and preferably air are used as the pressurized fluid.

The oils containing essential fatty acids or PUFAs are chosen, preferably, among grape seed, maize, rice, soybean, safflower, borage, Oenothera biennis (evening primrose), sunflower and blackcurrant oil.

Vitamin E may be used as d-α-tocopherol or as a mixture of the two d and 1 enantiomers of α-tocopherol or as a mixture of other tocopherols (β,χ,ε,ζ,η).

The composition for topical application contained in the device according to the invention can advantageously include other compounds having antioxidant activity, such as lipoic acid, coenzyme Q₁₀, vitamin A and derivatives thereof, in particular retinal and retinol palmitate and fat-soluble derivative of vitamin C, in particular ascorbyl palmitate.

Another compound that can advantageously be included in the composition according to the invention is lecithin, in particular soy lecithin. Lecithin represents a source of phospholipids which readily integrate in the membranes of the epidermal cells, giving rise to an increase of the skin elasticity and plasticity. Moreover, lecithin contributes to the stabilization of the overall composition, by virtue of its emulsifying action.

If desired, a fragrance may be added to the composition.

The composition contained in the device according to the invention may also serve as vehicle for various pharmaceutical or cosmetic active principles of lipophilic nature, for which it enhances the absorption through the skin.

Should it be desirable to use the device according to the present invention in the prevention of erythema, photodermatitis and sunburns due to exposure to ultraviolet rays (sun or artificial sources) or to ionizing radiation, the composition therein contained may comprise other compounds provided with absorption properties in the field of ultraviolet radiation, such as miroxil, salicylates, p-aminobenzoates, benzophenones, cinnamates, derivatives of dibenzoylmethane, derivatives of benzylidenecamphor, naphthoates, gallates and mixtures thereof, or physical filters, such as for example titanium dioxide, zinc oxide, barium sulfate, calcium carbonate and magnesium carbonate.

The composition contained in the device according to the invention is preferably the one reported hereinbelow (the percentages are by weight on the total weight of the composition):

| | |
|---|---|
| Vitamin E | 5-30 % |
| Oil rich in EFAs and/or PUFAs | 4-30 % |
| Ascorbyl palmitate | 0,2-3 % |
| Coenzyme Q₁₀ | 0,01-2 % |
| Lipoic acid | 0,01-2 % |
| Retinol palmitate | 0,01-2 % |
| Lecithin | 0-2 % |
| Pentamer cyclomethicone | q.s. to 100 % |

As used herein, the expression "oil rich in EFAs and/or PUFAs" means an oil having a content of essential fatty acids (EFA) and/or of Ω-6 or Ω-3 polyunsaturated fatty acids of at least 50 % in weight.

The filling of the collapsible container with the above reported composition can be advantageously carried out under a nitrogen atmosphere, in order to ensure the absence of air inside the container and accordingly absolutely prevent any possible phenomenon of oxidation involving the active principles of the composition.

EP-A-0 711 540 discloses an anti-againg cream comprising vitamin E, blackcormnt oil and a volatile silicone oil with the registered trade mark "Dow Corning Fluid 345®".

Spray dispensing devices made up of a bomb provided inside it with a collapsible container are for example described in patent application FR 2 436 085.

It has been found as particularly convenient for the purposes of the present invention to use a spray device marketed by the firm EP SPRAY SYSTEM SA (Switzerland).

By making use of the device according to the present invention, it is possible to apply the composition in a finely atomized way, thus forming a film endowed with properties of remarkable bio-adhesiveness with respect to the skin, whereby an immediate and effective absorption of the active principles therein contained is promoted. All this occurs not only thanks to the action of the atomizer, but, also and above all, thanks to the specific properties of the vehicle.

However, the most important advantage achieved by the device according to the present invention consists in the possibility of maintaining throughout the whole period of storage of the composition (at least two years) the integrity of the active principles, vitamin E and essential fatty acids, by the device protecting such active principles from oxidations and degradations caused by atmospheric oxygen and by light. Vitamin E in particular, kept aside from entering into contact with air and light, is capable of carrying out at its best its antioxidant action and of preventing the EFA and PUFA degradation.

The presence, together with tocopherol, of the above-mentioned other natural compounds having an antioxidant activity, further increases the stability of the composition of the device according to the invention, thanks to the synergistic action brought about by such compounds with tocopherol in preventing the oxidation of the essential fatty acids. Furthermore, the great amount of tocopherol has a protective effect with respect to the other antioxidant substances contained in the composition, allowing them to fully carry out for a long time their typical biological action.

Finally, another advantage of the device according to the invention consists in the absence of preservatives in the composition.

Further advantages of the device according to the invention will become apparent from the examples hereinbelow, which are reported as a matter of non-limiting illustration only, whereby reference is made to the only schematic drawing annexed.

In such a drawing, a device according to the invention is schematically shown, which is comprised of a collapsible container 1, which contains the above described composition, connected to a valve 2 onto which a sprayhead 3 acts; the collapsible container 1 is in turn enclosed inside a bomb 4, with valve 2 cramped to the lip of the bomb 4, and the gap between the inner wall of the bomb 4 and the container 1 is filled with a pressurized gas.

### EXAMPLE 1

| | |
|---|---|
| Vitamin E | 20 % |
| Grape seed oil | 10 % |
| Ascorbyl palmitate | 1 % |
| Coenzyme Q₁₀ | 0,1 % |
| Lipoic acid | 0,1 % |
| Retinol palmitate | 0,1 % |
| Pentamer cyclomethicone | q.s. to 100 % |

After having inserted the collapsible container 1 into bomb 2 and cramped the valve 2 to the lip of the bomb 4, the gap between the container 1 and the inner wall of the bomb 4 was filled with air at 10 atmospheres. Then, the solution obtained by dispersing in cyclomethicone the other above-listed compounds was introduced into the collapsible container 1 under a flow of nitrogen , according to methods well known in the art.

### EXAMPLE 2

| | |
|---|---|
| Vitamin E | 13 % |
| Grape seed oil | 5 % |
| Ascorbyl palmitate | 1 % |
| Coenzyme Q₁₀ | 0,1 % |
| Lipoic acid | 0,1 % |
| Retinol palmitate | 0,1 % |
| Pentamer cyclomethicone | q.s. to 100 % |

The above-reported composition was prepared as for Example 1 and the filling of the device was carried out in the same way as for Example 1.

### EXAMPLE 3

| | |
|---|---|
| Vitamin E | 14 % |
| Grape seed oil | 8 % |
| Butylmethoxydibenzoylmethane | 5 % |
| Ascorbyl palmitate | 1 % |
| Coenzyme Q₁₀ | 0,1 % |
| Lipoic acid | 0,1 % |
| Retinol palmitate | 0,1 % |
| Pentamer cyclomethicone | q.s. to 100 % |

The above reported composition is particularly well suited for the prevention of erythema and sunburns due to exposure to the sun or to other sources of UV radiation. With respect to conventional sun-oil and sun-creams formulations, the above reported one, besides the shield action with respect to UV radiation, also has an emollient, moisturizing and protective action for the skin not only with respect to UV radiation but also to other physical-chemical or atmospheric agents. Moreover, it is able to slow down the cellular aging processes, which are on the contrary accelerated by the skin exposure to sun radiation or UV radiation in general. In fact, thanks to its antioxidant action, the vitamin E contained in the composition exerts an action of sun filter for UVB radiation and protects the cells of the epidermis and dermis from the damages due to repeated overexposure to UVA radiation (photoageing and photocarcinogenesis).

### EXAMPLE 4

| | |
|---|---|
| Vitamin E | 20 % |
| Grape seed oil | 7 % |
| APEROXID TLA® | 2% |
| Ascorbyl palmitate | 1 % |
| Coenzyme Q₁₀ | 0,1 % |
| Lipoic acid | 0,1 % |
| Retinol palmitate | 0,1 % |
| Pentamer cyclomethicone | q.s. to 100 % |

APEROXID TLA® is a product marketed by BIOCHIM S.r.l. (Italy), which contains more than 50% b.w. of soy lecithin, 10-15% of mixed natural tocopherols, 8-10% ascorbyl palmitate and 1-5% citric acid.

The lecithin phospholipids contained in this formulation are easily integrated in the membranes of the epidermal cells and bring about a further increase of the elasticity and plasticity of the skin.

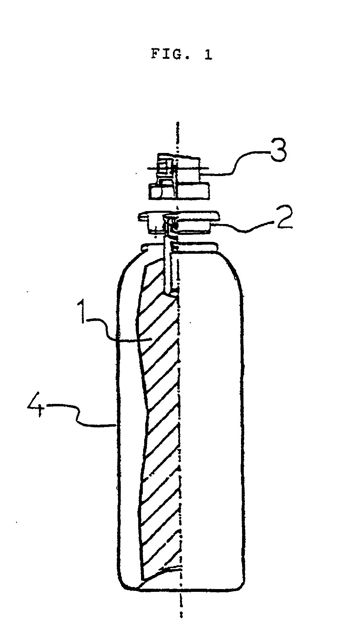

## Claims

1. Use of a device comprising a collapsible container (1) in turn enclosed in a spray bomb (4) filled with a pressurized fluid for spray dispensing a composition for topical application in finely atomized form, wherein said composition comprises:
- a low vapor pressure vehicle consisting of one or more volatile silicones,
- at least one component chosen among essential fatty acids, polyunsaturated Ω-6 or Ω-3 fatty acids and oils that contain them and
- vitamin E.

2. The use according to claim 1, wherein said one or more volatile silicones are chosen among pentamer cyclomethicone, tetramer cyclomethicone, hexamethyldisiloxane and mixtures thereof.

3. The use according to claim 2, wherein said pressurized fluid is chosen from the group comprising of air, carbon dioxide and nitrogen.

4. The use according to any one of the previous claims wherein said oils containing essential fatty acids or polyunsaturated Ω-6 or Ω-3 fatty acids are selected among grape seed, maize, rice, soybean, safflower, borage, Oenothera biennis, sunflower and blackcurrant oil.

5. The use according to any one of the previous claims wherein said composition further comprises one or more compounds selected from the group consisting of lipoic acid, coenzyme Q₁₀, vitamin A and derivatives thereof, lecithin and liposoluble derivatives of vitamin C.

6. The use according to any one of the previous claims wherein said composition further comprises compounds provided with absorption properties in the range of ultraviolet radiation selected from the group comprising miroxil, salicylates, p-aminobenzoates, benzophenones, cinnamates, derivatives of dibenzoylmethane, derivatives of benzylidenecamphor, naphthoates, gallates and mixtures thereof, and/or physical filters, selected from the group comprising titanium dioxide, zinc oxide, barium sulfate, calcium carbonate and magnesium carbonate.

7. The use according to any one of claims 1-5, wherein said composition is as follows:
| | |
|---|---|
| Vitamin E | 5-30 % |
| Oil rich in EFA and/or PUFA | 4-30 % |
| Ascorbyl palmitate | 0,2-3 % |
| Coenzyme Q₁₀ | 0,01-2 % |
| Lipoic acid | 0,01-2 % |
| Retinol palmitate | 0,01-2 % |
| Lecithin | 0-2% |
| Pentamer cyclomethicone | q.s. to 100 % |

8. The use according to any one of claims 1 to 5 and 7, wherein said composition further comprises at least one pharmaceutically active substance.

## Patentansprüche

1. Verwendung einer Vorrichtung, die einen zusammenlegbaren Behälter (1) umfasst, der wiederum in einer Spraydose (4) eingeschlossen ist, welche mit einer unter Druck stehenden Flüssigkeit zum Zerstäuben einer Zusammensetzung für die topische Anwendung in fein zerstäubter Form gefüllt ist, wobei die Zusammensetzung folgendes umfasst:
- einen Niederdampfdruck-Träger, der aus einem oder mehreren flüchtigen Silikonen besteht;
- mindestens eine Komponente, die aus den essentiellen Fettsäuren, mehrfach ungesättigten Ω-6- und Ω-3-Fettsäuren und diese enthaltenden Ölen ausgewählt ist, und
- Vitamin E.

2. Verwendung nach Anspruch 1, wobei die ein oder mehreren flüchtigen Silikone aus Pentamercyclomethicon, Tetramercyclomethicon, Hexamethyldisiloxan und Gemischen davon ausgewählt sind.

3. Verwendung nach Anspruch 2, wobei die unter Druck stehende Flüssigkeit aus der Gruppe ausgewählt ist, die Luft, Kohlendioxid und Stickstoff umfasst.

4. Verwendung nach einem der vorhergehenden Ansprüche, wobei die Öle, die essentielle Fettsäuren oder mehrfach ungesättigte Ω-6- oder Ω-3-Fettsäuren enthalten, aus Weintraubenkern-, Mais-, Reis-, Soja-, Saflor-, Borretsch-, Oenothera biennis-, Sonnenblumenund schwarzem Johannesbeeröl ausgewählt sind.

5. Verwendung nach einem der vorhergehenden Ansprüche, wobei die Zusammensetzung weiter eine oder mehrere Verbindungen umfasst, die aus der Gruppe bestehend aus Liponsäure, Coenzym Q₁₀, Vitamin A und Derivaten davon, Lecithin und fettlöslichen Derivaten von Vitamin C ausgewählt sind.

6. Verwendung nach einem der vorhergehenden Ansprüche, wobei die Zusammensetzung weiter Verbindungen umfaßt, die im Bereich der Ultraviolettstrahlung mit Absorptionseigenschaften ausgestattet sind und aus der Miroxil, Salicylate, p-Aminobenzoate, Benzophenone, Cinnamate, Derivate von Dibenzoylmethan, Derivate von Benzylidencampher, Naphthoate, Gallate und Gemische davon umfassenden Gruppe, ausgewählt sind, und/oder physikalische Filter umfasst, die aus der Titandioxid, Zinkoxid, Bariumsulfat, Calciumcarbonat und Magnesiumcarbonat umfassenden Gruppe ausgewählt sind.

7. Verwendung nach einem der Ansprüche 1 bis 5, wobei die Zusammensetzung wie folgt ist:
| | |
|---|---|
| Vitamin E | 5-30 % |
| EFA- und/oder PUFA-reiches Öl | 4-30 % |
| Ascorbylpalmitat | 0,2-3 % |
| Coenzym Q₁₀ | 0,01-2 % |
| Liponsäure | 0,01-2 % |
| Retinolpalmitat | 0,01-2 % |
| Lecithin | 0-2 % |
| Pentamercyclomethicon | auf 100 % |

8. Verwendung nach einem der Ansprüche 1 bis 5 und 7, wobei die Zusammensetzung weiter mindestens eine pharmazeutisch aktive Substanz umfasst.

## Revendications

1. Utilisation d'un dispositif comprenant un récipient écrasable (1) lui-même enfermé dans un bombe pulvérisatrice (4) remplie d'un fluide pressurisé pour délivrer par pulvérisation une composition pour application topique sous forme finement atomisée, où ladite composition comprend :
- un véhicule à faible pression de vapeur consistant en un ou plusieurs silicones volatils,
- au moins un composant choisi parmi les acides gras essentiels, les acides gras Q-6 ou Ω-3 polyinsaturés et les huiles qui les contiennent, et
- de la vitamine E.

2. L'utilisation selon la revendication 1, dans laquelle le(s)dit(s) un ou plusieurs silicones sont choisis parmi le cyclométhicone pentamère, le cyclométhicone tétramère, l'hexaméthyldisiloxane et leurs mélanges.

3. L'utilisation selon la revendication 2, dans laquelle ledit fluide pressurisé est choisi dans le groupe comprenant l'air, le dioxyde de carbone et l'azote.

4. L'utilisation selon l'une des revendications précédentes, dans laquelle lesdites huiles contenant des acides gras essentiels ou des acides gras Ω-6 ou Ω-3 polyinsaturés sont choisies parmi l'huile de pépin de raisin, de maïs, de riz, de soja, de carthame, de bourrache, d'Oenothera biennis, de tournesol et de cassis.

5. L'utilisation selon l'une des revendications précédentes, dans laquelle ladite composition comprend en outre un ou plusieurs composés choisis dans le groupe formé par l'acide lipoïque, la coenzyme Q₁₀, la vitamine A et ses dérivés, la lécithine et les dérivés liposolubles de la vitamine C.

6. L'utilisation selon l'une des revendications précédentes, dans laquelle ladite composition comprend en outre des composés présentant des propriété d'absorption dans le domaine du rayonnement ultraviolet choisis dans le groupe comprenant le miroxil, les salicylates, les p-aminobenzoates, les benzophénones, les cinnamates, les dérivés du dibenzoylméthane, les dérivés du benzylidènecamphre, les naphtoates, les gallates et leurs mélanges, et/ou des filtre physiques choisis dans le groupe comprenant le dioxyde de titane, l'oxyde de zinc, le sulfate de baryum, le carbonate de calcium et le carbonate de magnésium.

7. L'utilisation selon l'une des revendications 1 à 5, dans laquelle ladite composition est la suivante :
| | |
|---|---|
| Vitamine E | 5 à 30 % |
| Huile riche en AGE et/ou AGPI | 4 à 30 % |
| Palmitate d'ascorbyle | 0,2 à 3 % |
| Coenzyme Q₁₀ | 0,01 à 2 % |
| Acide lipoïque | 0,01 à 2 % |
| Palmitate de rétinol | 0,01 à 2 % |
| Lécithine | 0 à 2 % |
| Cyclométhicone pentamère | q.s. 100 % |

8. L'utilisation selon l'une des revendications 1 à 5 et 7, dans laquelle ladite composition comprend en outre au moins une substance pharmaceutiquement active.
